# EUROPEAN PATENT SPECIFICATION

(11) **EP 3 570 852 B1**
(45) Date of publication and mention of the grant of the patent: **30.07.2025**
(21) Application number: 18741137.6
(22) Date of filing: 19.01.2018
(51) Int. Cl.: A61K 35/17, A61P 25/00, A61P 25/28, G01N 33/564, C12Q 1/00, A61K 35/12, A61K 39/00, A61K 39/12, C07K 14/525, C07K 14/55, C07K 14/56, C07K 14/705, C12N 5/0783

(54) **AUTOLOGOUS CYTOTOXIC T CELLS (CTLS) EXPRESSING AN EBV-BINDING T CELL RECEPTOR FOR USE IN TREATING OR PREVENTING PRIMARY PROGRESSIVE MULTIPLE SCLEROSIS (MS) OR SECONDARY PROGRESSIVE MS**
AUTOLOGISCHE CYTOTOXISCHE T-ZELLEN (CTLS), DIE EINEN EBV-BINDENDEN T-ZELL-REZEPTOR EXPRIMIEREN, ZUR VERWENDUNG BEI DER BEHANDLUNG ODER VORBEUGUNG VON PRIMÄR PROGREDIENTER MULTIPLER SKLEROSE (MS) ODER SEKUNDÄR PROGREDIENTER MS
LYMPHOCYTES T CYTOTOXIQUES AUTOLOGUES (LTC) EXPRIMANT UN RÉCEPTEUR DE LYMPHOCYTES T LIÉS À L'EBV POUR UNE UTILISATION DANS LE TRAITEMENT OU LA PRÉVENTION DE LA SCLÉROSE EN PLAQUES (SEP) PROGRESSIVE PRIMAIRE OU DE LA SEP PROGRESSIVE SECONDAIRE

(30) Priority: 20.01.2017 US 201762448707 P; 24.10.2017 US 201762576349 P
(43) Date of publication of application: 27.11.2019
(73) Proprietor: Atara Biotherapeutics, Inc., Thousand Oaks, CA 91320 (US); The Council of the Queensland Institute of Medical Research, Herston, Queensland 4006 (AU)
(72) Inventor: KHANNA, Rajiv, Herston QLD 4006 (AU); SMITH, Corey, Ashgrove QLD 4060 (AU); AFTAB, Blake, Tolu, Thousand Oaks CA 91320 (US)
(74) Representative: Vossius & Partner Patentanwälte Rechtsanwälte mbB
(86) International application number: PCT/US2018/014458
(87) International publication number: WO 2018/136762

(56) References cited:
- WO-A1-2016/191756
- WO-A1-2017/203356
- WO-A1-2017/203368
- WO-A1-99/13904
- WO-A2-2004/015070
- US-A1- 2014 044 687
- C. SMITH ET AL: "Functional Reversion of Antigen-Specific CD8+ T Cells from Patients with Hodgkin Lymphoma following In Vitro Stimulation with Recombinant Polyepitope", THE JOURNAL OF IMMUNOLOGY, vol. 177, no. 7, 1 October 2006 (2006-10-01), US, pages 4897 - 4906, XP055310744, ISSN: 0022-1767, DOI: 10.4049/jimmunol.177.7.4897
- VOETEN J T ET AL: "Antigen processing for MHC class I restricted presentation of exogenous influenza A virus nucleoprotein cells", CLINICAL AND EXPERIMENTAL IMMUNOLOGY, WILEY-BLACKWELL PUBLISHING LTD, GB, vol. 125, no. 3, 1 September 2001 (2001-09-01), pages 423 - 431, XP003021171, ISSN: 0009-9104, DOI: 10.1046/J.1365-2249.2001.01613.X
- PENDER, M.P. ET AL.: "Epstein-Barr virus and multiple sclerosis: potential opportunities for immunotherapy", CLIN TRANSL IMMUNOLOGY, vol. 3, no. 10, 31 October 2014 (2014-10-31), pages e27, XP055445185
- PENDER M.P. ET AL.: "Epstein-Barr virus-specific adoptive immunotherapy for progressive multiple sclerosis", J. MULT SCLER., vol. 20, no. 11, October 2014 (2014-10-01), pages 1541 - 1544, XP055445181
- PENDER M.P. ET AL.: "Epstein-Barr virus-specific adoptive immunotherapy: a new horizon for multiple sclerosis treatment?", IMMUNOTHERAPY, vol. 6, no. 6, 2014, pages 659 - 661, XP055445188
- SMITH C. ET AL.: "Effective treatment of metastatic forms of Epstein-Barr virus-associated nasopharyngeal carcinoma with a novel adenovirus-based adoptive immunotherapy", CANCER RES., vol. 72, no. 5, 1 March 2012 (2012-03-01), pages 1116 - 1125, XP055148903
- JAQUIERY, E. ET AL.: "Intrathecal immune responses to EBV in early MS", EUR J IMMUNOL., vol. 40, no. 3, March 2010 (2010-03-01), pages 878 - 887, XP055504095
- LEGROUX, L. ET AL.: "Multiple Sclerosis and T Lymphocytes: An Entangled Story", J NEUROIMMUNE PHARMACOL., vol. 10, no. 4, December 2015 (2015-12-01), pages 528 - 546, XP035941002
- JILEK, S. ET AL.: "HLA-B7-restricted EBV-specific CD 8+ T cells are dysregulated in multiple sclerosis", J IMMUNOL., vol. 188, no. 9, 1 May 2012 (2012-05-01), pages 4671 - 4680, XP055504103
- BITSCH, A. ET AL.: "Differentiation of multiple sclerosis subtypes: implications for treatment", CNS DRUGS, vol. 16, no. 6, 2002, pages 405 - 18, XP9515683
- LELIC, A. ET AL.: "The Polyfunctionality of Human Memory CD 8+ T Cells Elicited by Acute and Chronic Virus Infections Is Not Influenced by Age", PLOS PATHOG., vol. 8, no. 12, December 2012 (2012-12-01), pages e1003076, XP055504107, [retrieved on 20121213]

## Description

### BACKGROUND

Autoimmune diseases, such as multiple sclerosis (MS), are pathologies arising from abnormal immune response against the body's own tissue. MS is characterized by the degradation of the myelin, a protective lipid shell surrounding nerve fibers, by the body's own immune cells.

Epstein Barr Virus (EBV), also known as human herpesvirus 4, is a ubiquitous herpes virus. Recently, it has been shown that exposure to EBV can predispose or otherwise play a role in the pathogenesis of autoimmune diseases, including MS. For example, recent studies have shown that individuals diagnosed with MS show higher levels of EBV related proteins in B cells aggregated in nerve tissue than healthy individuals. It is hypothesized that an increase of EBV-infected B cells and/or defective elimination of such cells may predispose individuals to multiple sclerosis.

### SUMMARY

The present invention is defined by the claims. Accordingly, the present invention relates to autologous cytotoxic T cells (CTLs) expressing a T cell receptor that specifically binds to an EBV peptide presented on a class I MHC for use in treating or preventing secondary progressive multiple sclerosis (MS) or primary progressive MS in a subject, wherein at least 30% of the CTLs express CD107a, IFNγ, TNFa, and IL-2.

In accordance with a preferred embodiment at least 40%, at least 50%, or at least 70% of the CTLs express IFNγ, CD107a, IL-2, and TNFa.

The present invention also relates to autologous cytotoxic T cells (CTLs) for use in treating or preventing secondary progressive multiple sclerosis (MS) or primary progressive MS in a subject, wherein the CTLs are prepared by a method comprising:
a) incubating a sample comprising autologous cytotoxic T cells (CTLs) with antigen-presenting cells (APCs) presenting an EBV peptide, thereby inducing proliferation of peptide-specific T cells in the sample; and
b) analyzing the expression of IFNγ, CD107a, IL-2, and TNFa by the proliferated peptide-specific autologous CTLs;
wherein the peptide-specific autologous CTLs are for administration to the subject if at least 30% of the peptide-specific autologous CTLs express CD107a, IFNγ, TNFa, and IL-2.

In accordance with a preferred embodiment the peptide-specific autologous CTLs are for administration to the subject if at least 40%, or at least 50% of the proliferated peptide-specific autologous CTLs express IFNγ, CD107a, IL-2, and TNFa.

In accordance with a further preferred embodiment the APCs comprise B cells, antigen-presenting T-cells, dendritic cells, artificial antigen-presenting cells, or aK562 cells.

In accordance with another preferred embodiment the EBV peptide comprises an amino acid sequence listed in Table 1.

In accordance with a preferred embodiment administering about 5 × 10⁶ CTLs, about 1 × 10⁷ CTLs, about 1.5 × 10⁷ CTLs, or about 2 × 10⁷ CTLs to the subject in a dose is comprised.

### BRIEF DESCRIPTION OF THE DRAWINGS

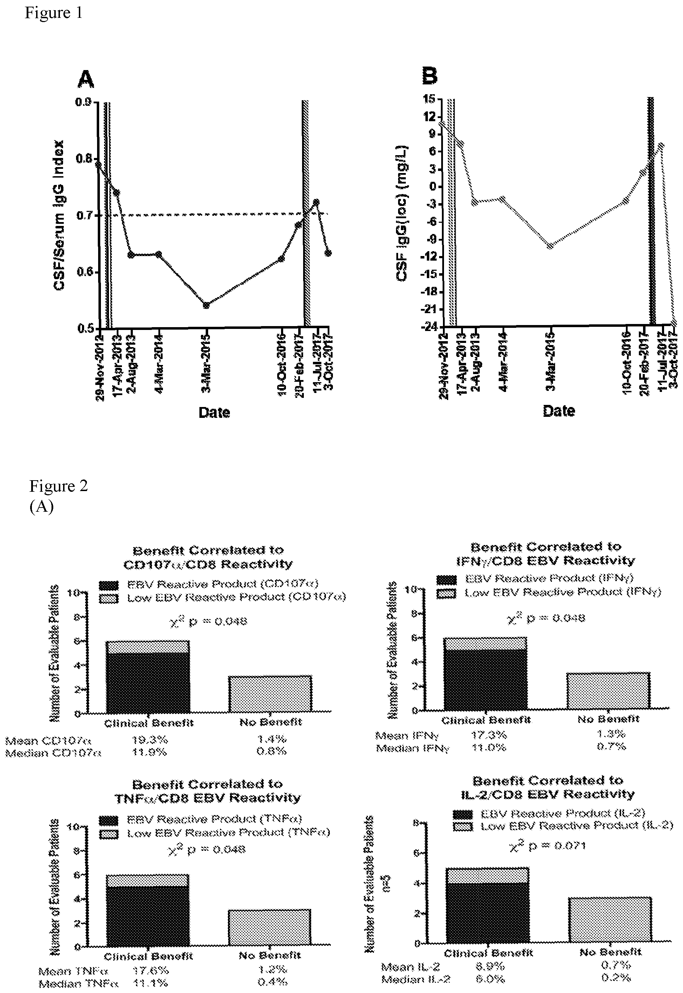

**Figure 1** has two panels (A-B), and shows CSF before and after autologous EBV-specific T cell therapy in the original patient after the first course of treatment 4 years ago and after retreatment this year in the current trial. (A) CSF IgG index, with dotted horizontal line indicating upper limit of normal range. (B) Intrathecal IgG production (IgG(loc)) was calculated by the formula of Reiber and Felgenhauer (ref. 16): IgG(loc) (mg/L) = {(CSF IgG ÷ serum IgG) - [0.8 × (√((CSF albumin ÷ serum albumin)2 + 15))] + 1.8} × serum IgG. Vertical lines indicate successive T cell infusions of 5 × 106, 1 × 107, 1.5 × 107 and 2 × 107 cells.
**Figure 2** has two parts (A-B), and shows correlations between EBV-specific CD8+ T cell reactivity of T cell product and clinical response to T cell therapy.
**Figure 3** shows disease activity on an MRI of the brain.

### DETAILED DESCRIPTION

### General

The present invention is defined by the claims. Also provided herein but not forming part of the claimed subject-matter are methods of treating multiple sclerosis (*e.g.,* relapsing-remitting MS, secondary progressive MS, primary progressive MS, or progressively relapsing MS) in a subject using autologous T cells (*e.g.,* CTLs) that recognize one or more EBV epitopes (*e.g.,* an EBV epitope disclosed herein). In some implementations, the method further comprises isolating sample comprising T cells from the subject, incubating the T cells with APCs presenting an EBV peptide (e.g., an EBV peptide disclosed herein), thereby generating T cells that recognize an EBV peptide presented on an MHC. Also provided herein are methods of assessing the efficacy of adoptive immunotherapy in a subject with multiple sclerosis, by obtaining samples of cerebral spinal fluid (CSF) from the subject both prior to and after T cells administration, and analyzing the relative amount of anti-EBV IgG in the CSF.

### Definitions

For convenience, certain terms employed in the specification, examples, and appended claims are collected here.

The articles "*a*" and "*an*" are used herein to refer to one or to more than one (*i.e., to* at least one) of the grammatical object of the article. By way of example, "an element" means one element or more than one element.

As used herein, the term *"administering"* means providing a pharmaceutical agent or composition to a subject, and includes, but is not limited to, administering by a medical professional and self-administering. Such an agent can contain, for example, peptide described herein, an antigen presenting cell provided herein and/or a T cell provided herein.

The term *"amino acid"* is intended to embrace all molecules, whether natural or synthetic, which include both an amino functionality and an acid functionality and capable of being included in a polymer of naturally-occurring amino acids. Exemplary amino acids include naturally-occurring amino acids; analogs, derivatives and congeners thereof; amino acid analogs having variant side chains; and all stereoisomers of any of any of the foregoing.

The term *"binding"* or *"interacting"* refers to an association, which may be a stable association, between two molecules, *e.g.,* between a TCR and a peptide/MHC, due to, for example, electrostatic, hydrophobic, ionic and/or hydrogen-bond interactions under physiological conditions.

The term *"biological sample," "tissue sample,"* or simply "*sample*" each refers to a collection of cells obtained from a tissue of a subject. The source of the tissue sample may be solid tissue, as from a fresh, frozen and/or preserved organ, tissue sample, biopsy, or aspirate; blood or any blood constituents, serum, blood; bodily fluids such as cerebral spinal fluid, amniotic fluid, peritoneal fluid or interstitial fluid, urine, saliva, stool, tears; or cells from any time in gestation or development of the subject.

As used herein, the term "*cytokine*" refers to any secreted polypeptide that affects the functions of cells and is a molecule which modulates interactions between cells in the immune, inflammatory or hematopoietic response. A cytokine includes, but is not limited to, monokines and lymphokines, regardless of which cells produce them. For instance, a monokine is generally referred to as being produced and secreted by a mononuclear cell, such as a macrophage and/or monocyte. Many other cells however also produce monokines, such as natural killer cells, fibroblasts, basophils, neutrophils, endothelial cells, brain astrocytes, bone marrow stromal cells, epidermal keratinocytes and B-lymphocytes. Lymphokines are generally referred to as being produced by lymphocyte cells. Examples of cytokines include, but are not limited to, Interleukin-1 (IL-1), Interleukin-2 (IL-2), Interleukin-6 (IL-6), Interleukin-8 (IL-8), Tumor Necrosis Factor-alpha (TNFα), and Tumor Necrosis Factor beta (TNFβ).

The term "*epitope*" means a protein determinant capable of specific binding to an antibody or TCR. Epitopes usually consist of chemically active surface groupings of molecules such as amino acids or sugar side chains. Certain epitopes can be defined by a particular sequence of amino acids to which an antibody is capable of binding.

As used herein, the phrase "*pharmaceutically acceptable*" refers to those agents, compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, the phrase "*pharmaceutically-acceptable carrier*" means a pharmaceutically-acceptable material, composition or vehicle, such as a liquid or solid filler, diluent, excipient, or solvent encapsulating material, involved in carrying or transporting an agent from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; and (22) other non-toxic compatible substances employed in pharmaceutical formulations.

The terms "*polynucleotide*", and "*nucleic acid*" are used interchangeably. They refer to a polymeric form of nucleotides of any length, either deoxyribonucleotides or ribonucleotides, or analogs thereof. Polynucleotides may have any three-dimensional structure, and may perform any function. The following are non-limiting examples of polynucleotides: coding or non-coding regions of a gene or gene fragment, loci (locus) defined from linkage analysis, exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs. If present, modifications to the nucleotide structure may be imparted before or after assembly of the polymer. A polynucleotide may be further modified, such as by conjugation with a labeling component. In all nucleic acid sequences provided herein, U nucleotides are interchangeable with T nucleotides.

As used herein, a therapeutic that "*prevents*" a condition refers to a compound that, when administered to a statistical sample prior to the onset of the disorder or condition, reduces the occurrence of the disorder or condition in the treated sample relative to an untreated control sample, or delays the onset or reduces the severity of one or more symptoms of the disorder or condition relative to the untreated control sample.

As used herein, "*specific binding*" refers to the ability of a TCR to bind to a peptide presented on an MHC (*e.g.,* class I MHC or class II MHC). Typically, a TCR specifically binds to its peptide/MHC with an affinity of at least a K_{D} of about 10⁻⁴ M or less, and binds to the predetermined antigen/binding partner with an affinity (as expressed by K_{D}) that is at least 10 fold less, at least 100 fold less or at least 1000 fold less than its affinity for binding to a non-specific and unrelated peptide/MHC complex (*e.g.,* one comprising a BSA peptide or a casein peptide).

As used herein, the term "*subject*" means a human or non-human animal selected for treatment or therapy.

The phrases "*therapeutically-effective amount*" and "*effective amount*" as used herein means the amount of an agent which is effective for producing the desired therapeutic effect in at least a sub-population of cells in a subject at a reasonable benefit/risk ratio applicable to any medical treatment.

As used herein, the term "*treating*" a disease in a subject or "treating" a subject having or suspected of having a disease refers to subjecting the subject to a pharmaceutical treatment, *e.g.,* the administration a CTL described herein, such that at least one symptom of the disease is decreased or prevented from worsening.

The term "*vector*" refers to the means by which a nucleic acid can be propagated and/or transferred between organisms, cells, or cellular components. Vectors include plasmids, viruses, bacteriophage, pro-viruses, phagemids, transposons, and artificial chromosomes, and the like, that may or may not be able to replicate autonomously or integrate into a chromosome of a host cell.

### Peptides

In certain aspects, also provided herein but not forming part of the claimed subject-matter are methods of treating multiple sclerosis (*e.g.,* relapsing-remitting MS, secondary progressive MS, primary progressive MS, or progressively relapsing MS) using autologous T cells (*e.g.,* CTLs) expressing TCRs that specifically bind to peptides comprising EBV epitopes presented on MHC (*e.g.,* class I MHC). In some implementations, provided herein are methods generating such autologous T cells, for example, by incubating a sample comprising T cells (*i.e.,* autologous T cells) with antigen-presenting cells (APCs) that present one or more of the EBV epitopes described herein (*e.g.,* APCs that present a peptide described herein comprising a EBV epitope on a class I MHC complex).

In some implementations, the peptides provided herein comprise a sequence of any EBV viral protein (*e.g.,* a sequence of at least 5, 6, 7, 8, 9, 10, 11, 12, 13, 14, 15, 16, 17, 18, 19 or 20 contiguous amino acids of any EBV protein). In some implementations, the peptides provided herein comprise no more than 25, 20, 19, 18, 17, 16, 15, 14, 13, 12, 11 or 10 contiguous amino acids of the EBV viral protein.

In some implementations, the peptides provided herein comprise a sequence of LMP1 (*e.g.,* a sequence of at least 5, 6, 7, 8, 9, 10, 11, 12, 13, 14, 15, 16, 17, 18, 19 or 20 contiguous amino acids of LMP1). In some implementations, the peptides provided herein comprise no more than 25, 20, 19, 18, 17, 16, 15, 14, 13, 12, 11 or 10 contiguous amino acids of LMP1. An exemplary LMP1 amino acid sequence is provided below (SEQ ID NO: 1):

1 mdldlergpp gprrpprgpp lssyialall llllallfwl yiimsnwtgg allvlyafal

61 mlviiiliif ifrrdllcpl galcllllmi tlllialwnl hgqalylgiv lfifgcllvl

121 giwvyfleil wrlgatiwql lafflaffld illliialyl qqnwwtllvd llwlllflai

181 liwmyyhgqr hsdehhhdds lphpqqatdd ssnhsdsnsn egrhhllvsg agdapplcsq

241 nlgapgggpd ngpqdpdntd dngpqdpdnt ddngphdplp qdpdntddng pqdpdntddn

301 gphdplphnp sdsagndggp pnlteevenk ggdrgppsmt dggggdphlp tlllgtsgsg

361 gddddphgpv qlsyyd

In some implementations, the peptides provided herein comprise a sequence of LMP2A *(e.g.,* a sequence of at least 5, 6, 7, 8, 9, 10, 11, 12, 13, 14, 15, 16, 17, 18, 19 or 20 contiguous amino acids of LMP2A). In some implementations, the peptides provided herein comprise no more than 25, 20, 19, 18, 17, 16, 15, 14, 13, 12, 11 or 10 contiguous amino acids of LMP2A. An exemplary LMP2A amino acid sequence is provided below (SEQ ID NO: 2):

1 mgslemvpmg agppspggdp dgddggnnsq ypsasgsdgn tptppndeer esneeppppy
61 edldwgngdr hsdyqplgnq dpslylglqh dgndglpppp ysprddssqh iyeeagrgsm
121 npvclpviva pylfwlaaia ascftasvst vvtatglals llllaavass yaaaqrkllt
181 pvtvltavvt ffaicltwri edppfnsllf allaaagglq giyvlvmlvl lilayrrrwr
241 rltvcggimf lacvlvlivd avlqlspllg avtvvsmtll llafvlwlss pgglgtlgaa
301 lltlaaalal laslilgtln lttmfllmll wtlvvllics scsscpltki llarlflyal

361 allllasali aggsilqtnf kslsstefip nlfcmllliv agilfilail tewgsgnrty

421 gpvfmclggl ltmvagavwl tvmtntllsa wiltagflif ligfalfgvi rccryccyyc

481 ltleseerpp tpyrntv

In some implementations, the peptides provided herein comprise a sequence of EBNA1 *(e.g.,* a sequence of at least 5, 6, 7, 8, 9, 10, 11, 12, 13, 14, 15, 16, 17, 18, 19 or 20 contiguous amino acids of EBNA1). In some implementations, the peptides provided herein comprise no more than 25, 20, 19, 18, 17, 16, 15, 14, 13, 12, 11 or 10 contiguous amino acids of EBNA1. An exemplary EBNA1 amino acid sequence is provided below (SEQ ID NO: 3):

1 pffhpvgead yfeylqeggp dgepdvppga ieqgpaddpg egpstgprgq gdggrrkkgg
61 wfgkhrgqgg snpkfeniae glrvllarsh vertteegtw vagvfvyggs ktslynlrrg
121 talaipqcrl tplsrlpfgm apgpgpqpgp lresivcyfm vflqthifae vlkdaikdlv
181 mtkpaptcni kvtvcsfddg vdlppwfppm vegaaaegdd gddgdeggdg degeegqe

In some implementations, the peptide comprises the sequence of an epitope listed in Table 1.

**Table 1: Exemplary EBV viral protein epitopes**

| **Epitope Sequence** | **HLA Restriction** | **SEQ ID NO.** |
|---|---|---|
| CLGGLLTMV | A*02 | 4 |
| FLYALALLL | A*02 | 5 |
| YLQQNWWTL | A*02, A*68, A*69 | 6 |
| YLLEMLWRL | A*02 | 7 |
| ALLVLYSFA | A*02 | 8 |
| LLSAWILTA | A*0203 | 9 |
| LTAGFLIFL | A*0206 | 10 |
| SSCSSCPLSKI | A*11 | 11 |
| PYLFWLAA | A*23, A*24, A*30 | 12 |
| TYGPVFMCL | A*24 | 13 |
| VMSNTLLSAW | A*25 | 14 |
| CPLSKILL | B*08 | 15 |
| RRRWRRLTV | B*27 | 16 |
| IEDPPFNSL | B*40 | 17 |
| IALYLQQNW | B*57, B*58 | 18 |
| MSNTLLSAW | B*58 | 19 |
| VLKDAIKDL | A*0203 | 20 |
| RPQKRPSCI | B*07 | 21 |
| IPQCRLTPL | B*07 | 22 |
| YNLRRGTAL | B*08 | 23 |
| HPVGEADYFEY | B*35 | 24 |
| LSRLPFGMA | B*57 | 25 |
| FVYGGSKTSL | Cw*03 | 26 |

In some implementations, the peptides provided herein comprise two or more of the EBV epitopes. In some implementations, the peptides provided herein comprise at least 2, 3, 4, 5, 6, 7, 8, 9, 10, 11, 12, 13, 14, 15, 16, 17, 18, 19 or 20 EBV epitopes. For example, in some implementations, the peptide provided herein comprises two or more of the EBV epitopes connected by linkers (*e.g.,* polypeptide linkers).

In some implementations, the sequence of the peptides comprise an EBV viral protein sequence except for 1 or more *(e.g.,* 1, 2, 3, 4, 5, 6, 7, 8, 9, 10 or more) conservative sequence modifications. As used herein, the term "conservative sequence modifications" is intended to refer to amino acid modifications that do not significantly affect or alter the interaction between a TCR and a peptide containing the amino acid sequence presented on an MHC. Such conservative modifications include amino acid substitutions, additions (*e.g.,* additions of amino acids to the N or C terminus of the peptide) and deletions (e*.g.,* deletions of amino acids from the N or C terminus of the peptide). Conservative amino acid substitutions are ones in which the amino acid residue is replaced with an amino acid residue having a similar side chain. Families of amino acid residues having similar side chains have been defined in the art. These families include amino acids with basic side chains (*e.g.,* lysine, arginine, histidine), acidic side chains (*e.g.,* aspartic acid, glutamic acid), uncharged polar side chains (*e.g.,* glycine, asparagine, glutamine, serine, threonine, tyrosine, cysteine, tryptophan), nonpolar side chains (*e.g.,* alanine, valine, leucine, isoleucine, proline, phenylalanine, methionine), beta-branched side chains (*e.g.,* threonine, valine, isoleucine) and aromatic side chains (*e.g.,* tyrosine, phenylalanine, tryptophan, histidine). Thus, one or more amino acid residues of the peptides described herein can be replaced with other amino acid residues from the same side chain family and the altered peptide can be tested for retention of TCR binding using methods known in the art. Modifications can be introduced into an antibody by standard techniques known in the art, such as site-directed mutagenesis and PCR-mediated mutagenesis.

In some implementations, the peptides provided herein comprise a sequence that is at least 80%, 85%, 90%, 95% or 100% identical to an EBV viral protein sequence (*e.g.,* the sequence of a fragment of an EBV viral protein). To determine the percent identity of two amino acid sequences, the sequences are aligned for optimal comparison purposes (*e.g.,* gaps can be introduced in one or both of a first and a second amino acid sequence for optimal alignment and non-identical sequences can be disregarded for comparison purposes). The amino acid residues at corresponding amino acid positions are then compared. When a position in the first sequence is occupied by the same amino acid residue as the corresponding position in the second sequence, then the molecules are identical at that position. The percent identity between the two sequences is a function of the number of identical positions shared by the sequences, taking into account the number of gaps, and the length of each gap, which need to be introduced for optimal alignment of the two sequences.

In some implementations, the peptide is chimeric or fusion peptide. As used herein, a "chimeric peptide" or "fusion peptide" comprises a peptide having a sequence provided herein linked to a distinct peptide having sequence to which it is not linked in nature. For example, the distinct peptide can be fused to the N-terminus or C-terminus of the peptide provided herein either directly, through a peptide bond, or indirectly through a chemical linker. In some implementations, the peptide of the provided herein is linked to another peptide comprising a distinct EBV epitopes. In some implementations, the peptide provided herein is linked to peptides comprising epitopes from other viral and/or infectious diseases.

A chimeric or fusion peptide provided herein can be produced by standard recombinant DNA techniques. For example, DNA fragments coding for the different peptide sequences are ligated together in-frame in accordance with conventional techniques, for example by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another implementation, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and re-amplified to generate a chimeric gene sequence (see, for example, Current Protocols in Molecular Biology, Ausubel et al., eds., John Wiley & Sons: 1992). Moreover, many expression vectors are commercially available that already encode a fusion moiety.

The peptides provided herein can be isolated from cells or tissue sources by an appropriate purification scheme using standard protein purification techniques, and can be produced by recombinant DNA techniques, and/or can be chemically synthesized using standard peptide synthesis techniques. The peptides described herein can be produced in prokaryotic or eukaryotic host cells by expression of nucleotides encoding a peptide(s) of the present disclosure. Alternatively, such peptides can be synthesized by chemical methods. Methods for expression of heterologous peptides in recombinant hosts, chemical synthesis of peptides, and *in vitro* translation are well known in the art and are described further in Maniatis et al., Molecular Cloning: A Laboratory Manual (1989), 2nd Ed., Cold Spring Harbor, N. Y.; Berger and Kimmel, Methods in Enzymology, Volume 152, Guide to Molecular Cloning Techniques (1987), Academic Press, Inc., San Diego, Calif.; Merrifield, J. (1969) J. Am. Chem. Soc. 91:501; Chaiken I. M. (1981) CRC Crit. Rev. Biochem. 11:255; Kaiser et al. (1989) Science 243:187; Merrifield, B. (1986) Science 232:342; Kent, S. B. H. (1988) Annu. Rev. Biochem. 57:957; and Offord, R. E. (1980) Semisynthetic Proteins, Wiley Publishing.

In certain aspects, provided herein are nucleic acid molecules encoding the peptides described herein. In some implementations, the nucleic acid molecule is a vector. In some implementations, the nucleic acid molecule is a viral vector, such as an adenovirus based expression vector, that comprises the nucleic acid molecules described herein. In some implementations, the vector provided herein encodes a plurality of epitopes provided herein *(e.g.,* as a polyepitope). In some implementations, the vector provided herein encodes at least 2, 3, 4, 5, 6, 7, 8, 9, 10, 11, 12, 13, 14, 15, 16, 17, 18, 19 or 20 epitopes provided herein (*e.g.,* epitopes provided in Table 1).

In some implementations, the vector is AdE1-LMPpoly. The AdE1-LMPpoly vector encodes a polyepitope of defined CTL epitopes from LMP1 and LMP2 fused to a Gly-Ala repeat-depleted EBNA1 sequence. The AdE1-LMPpoly vector is described, for example, in Smith et al., Cancer Research 72:1116 (2012); Duraiswamy et al., Cancer Research 64:1483-9 (2004); and Smith et al., J. Immunol 117:4897-906.

As used herein, the term "vector," refers to a nucleic acid molecule capable of transporting another nucleic acid to which it has been linked. One type of vector is a "plasmid", which refers to a circular double-stranded DNA loop into which additional DNA segments may be ligated. Another type of vector is a viral vector, wherein additional DNA segments may be ligated into the viral genome. Certain vectors are capable of autonomous replication in a host cell into which they are introduced (*e.g.,* bacterial vectors having a bacterial origin of replication, episomal mammalian vectors). Other vectors (*e.g.,* non-episomal mammalian vectors) can be integrated into the genome of a host cell upon introduction into the host cell, and thereby be replicated along with the host genome. Moreover, certain vectors are capable of directing the expression of genes. Such vectors are referred to herein as "recombinant expression vectors" (or simply, "expression vectors"). In some implementations, provided herein are nucleic acids operably linked to one or more regulatory sequences (*e.g.,* a promotor) in an expression vector. In some implementations, the cell transcribes the nucleic acid provided herein and thereby expresses a peptide described herein. The nucleic acid molecule can be integrated into the genome of the cell or it can be extrachromosomal.

In some implementations, provided herein are cells that contain a nucleic acid described herein *(e.g.,* a nucleic acid encoding a peptide described herein). The cell can be, for example, prokaryotic, eukaryotic, mammalian, avian, murine and/or human. In some implementations, the cell is a mammalian cell. In some implementations the cell is an APC (*e.g.* an antigen-presenting T cell, a dendritic cell, a B cell, or an aK562 cell). In the present methods, a nucleic acid described herein can be administered to the cell, for example, as nucleic acid without delivery vehicle, in combination with a delivery reagent. In some implementations, any nucleic acid delivery method known in the art can be used in the methods described herein. Suitable delivery reagents include, but are not limited to, *e.g.,* the Mirus Transit TKO lipophilic reagent; lipofectin; lipofectamine; cellfectin; polycations (*e.g.,* polylysine), atelocollagen, nanoplexes and liposomes. In some implementations of the methods described herein, liposomes are used to deliver a nucleic acid to a cell or subject. Liposomes suitable for use in the methods described herein can be formed from standard vesicle-forming lipids, which generally include neutral or negatively charged phospholipids and a sterol, such as cholesterol. The selection of lipids is generally guided by consideration of factors such as the desired liposome size and half-life of the liposomes in the blood stream. A variety of methods are known for preparing liposomes, for example, as described in Szoka et al. (1980), Ann. Rev. Biophys. Bioeng. 9:467; and U.S. Pat. Nos. 4,235,871, 4,501,728, 4,837,028, and 5,019,369.

### Autologous T cells

Also provided herein but not forming part of the claimed subject-matter are methods of treating multiple sclerosis (*e.g.,* relapsing-remitting MS, secondary progressive MS, primary progressive MS or progressively relapsing MS) by administering to the subject autologous T cells *(e.g.,* CTLs) expressing a T cell receptor that specifically binds to an EBV peptide presented on an MHC. In some implementations, the MHC is a class I MHC. In some implementations, the MHC is a class II MHC.

In some implementations, provided herein are APCs that present a peptide described herein *(e.g.,* a peptide comprising a LMP1, LMP2A, or EBNA1 epitope sequence). In some implementations the APCs are B cells, antigen presenting T-cells, dendritic cells, or artificial antigen-presenting cells *(e.g.,* aK562 cells).

Dendritic cells for use in the process may be prepared by taking PBMCs from a patient sample and adhering them to plastic. Generally the monocyte population sticks and all other cells can be washed off. The adherent population is then differentiated with IL-4 and GM-CSF to produce monocyte derived dendritic cells. These cells may be matured by the addition of IL-1β, IL-6, PGE-1 and TNF-α (which upregulates the important co-stimulatory molecules on the surface of the dendritic cell) and are then transduced with one or more of the peptides provided herein.

In some implementations, the APC is an artificial antigen-presenting cell, such as an aK562 cell. In some implementations, the artificial antigen-presenting cells are engineered to express CD80, CD83, 41BB-L, and/or CD86. Exemplary artificial antigen-presenting cells, including aK562 cells, are described U.S. Pat. Pub. No. 2003/0147869.

In certain aspects, provided herein are methods of generating APCs that present the one or more of the EBV epitopes described herein comprising contacting an APC with a peptide comprising a EBV epitope and/or with a nucleic acid encoding a EBV epitope. In some implementations, the APCs are irradiated. In some implementations, the APCs that present a peptide described herein (*e.g.,* a peptide comprising a LMP1, LMP2A, or EBNA1 epitope sequence). A cell presenting a peptide described herein can be produced by standard techniques known in the art. For example, a cell may be pulsed to encourage peptide uptake. In some implementations, the cells are transfected with a nucleic acid encoding a peptide provided herein. Provided herein are methods of producing antigen-presenting cells (APCs), comprising pulsing a cell with the peptides described herein. Exemplary examples of producing antigen presenting cells can be found in WO2013088114.

In some implementations, provided herein are T cells (*e.g.,* CD4 T cells and/or CD8 T cells) that express a TCR *(*e*.g.,* an αβ TCR or a γδ TCR) that recognizes a peptide described herein presented on an MHC. In some implementations, the T cell is a CD8 T cell (e.g., a CTL) that expresses a TCR that recognizes a peptide described herein presented on a class I MHC. In some implementations, the T cell is a CD4 T cell (e.g., a helper T cell) that recognizes a peptide described herein presented on a class II MHC.

In some implementations, provided herein are methods of generating, activating and/or inducing proliferation of T cells (*e.g.,* autologous CTLs) that recognize one or more of the EBV epitopes described herein. In some implementations, a sample comprising autologous T cells *(e.g.,* a PBMC sample) is incubated in culture with an APC provided herein *(*e.g., an APC that presents a peptide comprising an EBV epitope on a class I MHC complex). In some implementations, the APCs are autologous to the subject from whom the T cells were obtained. In some implementations, the APCs are not autologous to the subject from whom the T cells were obtained. In some implementations, the sample containing T cells are incubated 2 or more times with APCs provided herein. In some implementations, the T cells are incubated with the APCs in the presence of at least one cytokine. In some implementations, the cytokine is IL-4, IL-7 and/or IL-15. Exemplary methods for inducing proliferation of T cells using APCs are provided, for example, in U.S. Pat. Pub. No. 2015/0017723.

In some implementations, provided herein are compositions (*e.g.,* therapeutic compositions) comprising T cells and/or APCs provided herein used to treat and/or prevent an autoimmune disease in a subject by administering to the subject an effective amount of the composition. In some aspects, provided herein are methods of treating multiple sclerosis using a composition (*e.g.,* a pharmaceutical composition, such compositions comprising autologous CTLs). In some implementations, the composition includes a combination of multiple (*e.g.,* two or more) CTLs provided herein.

### Therapeutic Methods

In some implementations, also provided herein but not forming part of the claimed subject-matter are methods of treating MS *(*e.g., primary progressive MS) in a subject by administering to the subject autologous T cells (*e.g.,* autologous CTLs) provided herein. In some implementations, the MS is relapsing-remitting MS, secondary progressive MS, primary progressive MS or progressively relapsing MS. In some implementations, the autologous T cells are isolated from a peripheral mononuclear blood sample. Expression of biomarkers by the autologous T cells may be assessed by any suitable method, such as flow cytometry. In some implementations, the autologous T cells are stimulated by a vector comprising EBV viral peptides (e.g., AdE1-LMPpoly). In some implementations, the autologous T cells are stimulated by a viral vector and sorted via flow cytometry. For example, the autologous T cells may undergo surface staining according to the protocols exemplified in Example 2. In some implementations, the autologous T cells are incubated with one or more antibodies specific for CD107A, and subsequently sorted by flow cytometry. In some implementations, the autologous T cells are incubated with one or more antibodies that bind to intracellular cytokines, such as antibodies specific for IFNγ, IL-2, and/or TNF. In some implementations, the autologous T cells are incubated with antibodies for intracellular cytokines and subsequently sorted via flow cytometry.

In some aspects, provided herein are methods of selecting a subject for adoptive immunotherapy by obtaining a PMBC sample from the subject, isolating the autologous T cells, determining the EBV reactivity of the autologous T cells, and if at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 40%, 50%, 60%, 70% or 80% of the autologous T cells are EBV reactive, selecting the subject for adoptive immunotherapy.

In some aspects, provided herein are methods of selecting a subject for adoptive immunotherapy by obtaining a sample comprising T cells (e.g., CTLs) from the subject, isolating the autologous T cells, and determining the CD107A expression of the autologous T cells, and if at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 40%, 50%, 60%, 70% or 80% of the autologous T cells express CD107A, selecting the subject for adoptive immunotherapy.

In some aspects, provided herein are methods of selecting a subject for adoptive immunotherapy by obtaining a sample comprising T cells (e.g., CTLs) from the subject, isolating the autologous T cells, determining the IFNγ expression of the autologous T cells, and if at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 40%, 50%, 60%, 70% or 80% of the autologous T cells express IFNγ selecting the subject for adoptive immunotherapy.

In some aspects, provided herein are methods of selecting a subject for adoptive immunotherapy by obtaining a sample comprising T cells (e.g., CTLs) from the subject, isolating the autologous T cells, determining the TNF expression of the autologous T cells, and if at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 40%, 50%, 60%, 70% or 80% of the autologous T cells express TNF, selecting the subject for adoptive immunotherapy.

In some aspects, provided herein are methods of selecting a subject for adoptive immunotherapy by obtaining a sample comprising T cells (e.g., CTLs) from the subject, isolating the autologous T cells, determining the IL-2 expression of the autologous T cells, and if at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 40%, 50%, 60%, 70% or 80% of the autologous T cells express Il-2, selecting the subject for adoptive immunotherapy.

In some implementations, the methods further comprise obtaining a sample comprising the T cells from the subject (*e.g.,* obtaining a PBMC sample from the subject). In some implementations, the autologous T cells (e.g., CD4+ T cells or CD8+ T cells) are isolated form the sample. In some implementations, the sample is comprised mostly or completely of autologous T cells.

Provided herein are methods of treating or preventing multiple sclerosis (MS) in a subject, comprising administering to the subject autologous cytotoxic T cells (CTLs) expressing a T cell receptor that specifically binds to an EBV peptide presented on a class I MHC. In some implementations, at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 40%, 50%, 60%, 70% or 80% of the T cells (e.g., CTLs) in the sample express CD107A. In some implementations, at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 40%, 50%, 60%, 70% or 80% of the T cells (e.g., CTLs) in the sample express IFNy. In some implementations, at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 40%, 50%, 60%, 70% or 80% of the T cells (e.g., CTLs) in the sample express TNF. In some implementations, at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 40%, 50%, 60%, 70% or 80% of the T cells (e.g., CTLs) in the sample express IL-2.

In some implementations, at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80% 81%, 82%, 83%, 84%, 85%, 86%, 87%, 88%, 89%, or 90% of the T cells (e.g., CTLs) in the sample express CD107A and IFNγ.

In some implementations, at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80% 81%, 82%, 83%, 84%, 85%, 86%, 87%, 88%, 89%, or 90% of the T cells (e.g., CTLs) in the sample express CD107A and TNF.

In some implementations, at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80% 81%, 82%, 83%, 84%, 85%, 86%, 87%, 88%, 89%, or 90% of the T cells (e.g., CTLs) in the sample express CD107A and IL-2.

In some implementations, at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80% 81%, 82%, 83%, 84%, 85%, 86%, 87%, 88%, 89%, or 90% of the T cells (e.g., CTLs) in the sample express IFNy and TNF.

In some implementations, at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80% 81%, 82%, 83%, 84%, 85%, 86%, 87%, 88%, 89%, or 90% of the T cells (e.g., CTLs) in the sample express IFNy and IL-2.

In some implementations, at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80% 81%, 82%, 83%, 84%, 85%, 86%, 87%, 88%, 89%, or 90% of the T cells (e.g., CTLs) in the sample express TNF and IL-2.

In some implementations, at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80% 81%, 82%, 83%, 84%, 85%, 86%, 87%, 88%, 89%, or 90% of the T cells (e.g., CTLs) in the sample express IFNγ, TNF, and IL-2.

In some implementations, at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80% 81%, 82%, 83%, 84%, 85%, 86%, 87%, 88%, 89%, or 90% of the T cells (e.g., CTLs) in the sample express CD107A, TNF, and IL-2.

In some implementations, at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80% 81%, 82%, 83%, 84%, 85%, 86%, 87%, 88%, 89%, or 90% of the T cells (e.g., CTLs) in the sample express CD107A, IFNγ, and IL-2.

In some implementations, at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80% 81%, 82%, 83%, 84%, 85%, 86%, 87%, 88%, 89%, or 90% of the T cells (e.g., CTLs) in the sample express CD107A, IFNγ, and TNF.

In some implementations, at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80% 81%, 82%, 83%, 84%, 85%, 86%, 87%, 88%, 89%, or 90% of the T cells (e.g., CTLs) in the sample express CD107A, IFNγ, TNF, and IL-2.

In some implementations, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80% 81%, 82%, 83%, 84%, 85%, 86%, 87%, 88%, 89%, or 90% of the T cells (e.g., CTLs) are EBV reactive.

T cells biomarker expression and/or EBV reactivity may be measured and/or analyzed either before or after T cell expansion with a nucleic acid construct disclosed herein, polypeptide disclosed herein, or an APC.

In some aspects, provided herein are methods of treating or preventing MS in a subject by incubating antigen-presenting cells (APCs) with a nucleic acid construct encoding for an EBV peptide, thereby inducing the APCs to present an EBV peptide, inducing peptide-specific T cell (e.g., CTL) proliferation by incubating a sample comprising autologous T cells (e.g., CTLs) with the antigen-presenting cells (APCs), thereby inducing the autologous T cells (e.g., CTLs) to proliferate and administering the peptide-specific autologous T cells (e.g., CTLs) to the subject. In some implementations, EBV reactivity and biomarker expression is quantified prior to stimulation of the autologous T cells with a viral vector (e.g., a viral vector disclosed herein) and/or APCs (e.g., APCs disclosed herein). Alternatively or additionally, EBV reactivity and biomarker expression may be quantified after stimulation of the autologous T cells with a viral vector (e.g., a viral vector disclosed herein) and/or APCs (e.g., an APC transfected with a viral vector disclosed herein). In some implementations, EBV reactivity is measured by quantifying the percentage of T cells in the sample that express CD107A. In some implementations, EBV reactivity is measured by quantifying the percentage of T cells in the sample that express IFNy. In some implementations, EBV reactivity is measured by quantifying the percentage of T cells in the sample that express TNF. In some implementations, EBV reactivity is measured by quantifying the percentage of T cells in a sample that express IL-2. In some implementations, EBV reactivity is measured as a percentage of T cells that express multiple biomarkers (e.g., two or more of CD107A, IFNγ, TNF, and IL-2, preferably all four). In some implementations, the EBV reactivity is calculated by quantifying the percentage of autologous T cells in a sample that express CD107A, IFNγ, TNF, and IL-2. T cells may be isolated from a sample (e.g., a PBMC sample or a sample comprising T cells) either before or after EBV reactivity percentage quantification. Therefore, in some implementations, EBV reactivity is the percentage of T cells having the desired characteristic(s) in a sample that comprises mostly T cells.

In some implementations, EBV reactivity is measured by quantifying the percentage of CD8+ lymphocytes in the sample that express CD107A. In some implementations, EBV reactivity is measured by quantifying the percentage of CD8+ lymphocytes in the sample that express IFNy. In some implementations, EBV reactivity is measured by quantifying the percentage of CD8+ lymphocytes in the sample that express TNF. In some implementations, EBV reactivity is measured by quantifying the percentage of CD8+ lymphocytes in a sample that express IL-2. In some implementations, EBV reactivity is measured as a percentage of CD8+ lymphocytes that express multiple biomarkers (e.g., two or more of CD107A, IFNγ, TNF, and IL-2, preferably all four). CD8+ lymphocytes may be isolated from a sample (e.g., a PBMC sample or a sample of CD8+ lymphocytes) either before or after EBV reactivity percentage quantification. Therefore, in some implementations, EBV reactivity is the percentage of CD8+ lymphocytes having the desired characteristic(s) in a sample that comprises mostly or CD8+ lymphocytes.

In some implementations, EBV reactivity is measured by quantifying the percentage of CD3+ lymphocytes in the sample that express CD107A. In some implementations, EBV reactivity is measured by quantifying the percentage of CD3+ lymphocytes in the sample that express IFNy. In some implementations, EBV reactivity is measured by quantifying the percentage of CD3+ lymphocytes in the sample that express TNF. In some implementations, EBV reactivity is measured by quantifying the percentage of CD3+ lymphocytes in a sample that express IL-2. In some implementations, EBV reactivity is measured as a percentage of CD3+ lymphocytes that express multiple biomarkers (e.g., two or more of CD107A, IFNγ, TNF, and IL-2, preferably all four). CD3+ lymphocytes may be isolated from a sample (e.g., a PBMC sample or a sample of CD3+ lymphocytes) either before or after EBV reactivity percentage quantification. Therefore, in some implementations, EBV reactivity is the percentage of CD3+ lymphocytes having the desired characteristic(s) in a sample that comprises mostly CD3+ lymphocytes.

In some implementations, the method further comprises analyzing the expression of CD107a, IFNγ, TNF, or IL-2 by the proliferated peptide-specific autologous T cells (e.g., CTLs), and if at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80% 81%, 82%, 83%, 84%, 85%, 86%, 87%, 88%, 89%, or 90% of the proliferated peptide-specific autologous T cells (e.g., CTLs) express CD107a, IFNγ, TNF, or IL-2, administering the peptide-specific autologous T cells (e.g., CTLs) to the subject.

In some implementations, the method further comprises analyzing the expression of CD107a and TNF by the proliferated peptide-specific autologous T cells (e.g., CTLs), and if at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80% 81%, 82%, 83%, 84%, 85%, 86%, 87%, 88%, 89%, or 90% of the proliferated peptide-specific autologous T cells (e.g., CTLs) express CD107a and TNF, administering the peptide-specific autologous T cells (e.g., CTLs) to the subject.

In some implementations, the method further comprises analyzing the expression of CD107a and IFNy by the proliferated peptide-specific autologous T cells (e.g., CTLs), and if at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80% 81%, 82%, 83%, 84%, 85%, 86%, 87%, 88%, 89%, or 90% of the proliferated peptide-specific autologous T cells (e.g., CTLs) express CD107a and IFNγ, administering the peptide-specific autologous T cells (e.g., CTLs) to the subject.

In some implementations, the method further comprises analyzing the expression of CD107a and IL-2 by the proliferated peptide-specific autologous T cells (e.g., CTLs), and if at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80% 81%, 82%, 83%, 84%, 85%, 86%, 87%, 88%, 89%, or 90% of the proliferated peptide-specific autologous T cells (e.g., CTLs) express CD107a and IL-2, administering the peptide-specific autologous T cells (e.g., CTLs) to the subject.

In some implementations, the method further comprises analyzing the expression of TNF and IL-2 by the proliferated peptide-specific autologous T cells (e.g., CTLs), and if at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80% 81%, 82%, 83%, 84%, 85%, 86%, 87%, 88%, 89%, or 90% of the proliferated peptide-specific autologous T cells (e.g., CTLs) express TNF and IL-2, administering the peptide-specific autologous T cells (e.g., CTLs) to the subject.

In some implementations, the method further comprises analyzing the expression of IFNy and IL-2 by the proliferated peptide-specific autologous T cells (e.g., CTLs), and if at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80% 81%, 82%, 83%, 84%, 85%, 86%, 87%, 88%, 89%, or 90% of the proliferated peptide-specific autologous T cells (e.g., CTLs) express IFNγ and IL-2, administering the peptide-specific autologous T cells (e.g., CTLs) to the subject.

In some implementations, the method further comprises analyzing the expression of IFNy and TNF by the proliferated peptide-specific autologous T cells (e.g., CTLs), and if at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80% 81%, 82%, 83%, 84%, 85%, 86%, 87%, 88%, 89%, or 90% of the proliferated peptide-specific autologous T cells (e.g., CTLs) express IFNγ and TNF, administering the peptide-specific autologous T cells (e.g., CTLs) to the subject.

In some implementations, the method further comprises analyzing the expression of CD107a, IFNγ, and TNF by the proliferated peptide-specific autologous T cells (e.g., CTLs), and if at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80% 81%, 82%, 83%, 84%, 85%, 86%, 87%, 88%, 89%, or 90% of the proliferated peptide-specific autologous T cells (e.g., CTLs) express CD107a, IFNγ, and TNF, administering the peptide-specific autologous T cells (e.g., CTLs) to the subject.

In some implementations, the method further comprises analyzing the expression of CD107a, IFNγ, and IL-2 by the proliferated peptide-specific autologous T cells (e.g., CTLs), and if at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80% 81%, 82%, 83%, 84%, 85%, 86%, 87%, 88%, 89%, or 90% of the proliferated peptide-specific autologous T cells (e.g., CTLs) express CD107a, IFNγ, and IL-2, administering the peptide-specific autologous T cells (e.g., CTLs) to the subject.

In some implementations, the method further comprises analyzing the expression of CD107a, IL-2, and TNF by the proliferated peptide-specific autologous T cells (e.g., CTLs), and if at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80% 81%, 82%, 83%, 84%, 85%, 86%, 87%, 88%, 89%, or 90% of the proliferated peptide-specific autologous T cells (e.g., CTLs) express CD107a, IL-2, and TNF, administering the peptide-specific autologous T cells (e.g., CTLs) to the subject.

In some implementations, the method further comprises analyzing the expression of IFNγ, IL-2, and TNF by the proliferated peptide-specific autologous T cells (e.g., CTLs), and if at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80% 81%, 82%, 83%, 84%, 85%, 86%, 87%, 88%, 89%, or 90% of the proliferated peptide-specific autologous T cells (e.g., CTLs) express IFNγ, IL-2, and TNF, administering the peptide-specific autologous T cells (e.g., CTLs) to the subject.

In some implementations, if at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80% 81%, 82%, 83%, 84%, 85%, 86%, 87%, 88%, 89%, or 90% of the peptide-specific autologous T cells (e.g., CTLs) express CD107a, IFNγ, TNF, and IL-2, the autologous T cells (e.g., CTLs) are administered to the subject.

The peptide-specific autologous T cells (e.g., CTLs) may have at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80% 81%, 82%, 83%, 84%, 85%, 86%, 87%, 88%, 89%, or 90% of the peptide-specific autologous T cells (e.g., CTLs) have EBV reactivity.

In some implementations, about 1 × 10⁵ to about 1 × 10⁸ T- cells are administered to the subject per dose of T cells. In some implementations, about 1 × 10⁶ to about 1 × 10⁷ T cells are administered to the subject per dose of T cells. In some implementations, 5 × 10⁶, 1 × 10⁷, 1.5 × 10⁷, or 2 × 10⁷ T cells (e.g., CTLs) are administered to the subject. Multiple doses may be administered to the subject. In some implementations, an initial dose of T cells (e.g., autologous CTLs) is administered, and one or more additional doses of T cells (e.g., autologous CTLs) are administered, e.g., at increasing doses along the course of therapy. In some implementations, two or more, three or more, four or more, five or more, six or more, seven or more, eight or more, nine or more, or ten or more doses are administered. The subject may be administered additional doses that are the same or different from the initial dose. For example, a lower dose may be administered followed by a higher dose. The doses may be administered daily, twice a week, weekly, biweekly, once a month, once every two months, once every three months, or once every six months. In some implementations, the subject does not experience any adverse effects as a result of T cell (e.g., autologous CTL) administration.

In some aspects, the method further comprises assessing the efficacy of adoptive immunotherapy in a subject with multiple sclerosis, by obtaining a first sample of cerebral spinal fluid (CSF) from the subject, analyzing the amount of anti-EBV IgG in the CSF in the first sample (preferably before a CTL administration) and, after a period of time, obtaining a second sample of CSF from the subject (preferably after a CTL administration), analyzing the amount of anti-EBV IgG in the CSF in the second sample, and if the amount of anti-EBV IgG in the second sample is less than the first sample, the disease has stabilized and/or not progressed. Additional samples of CTF may be obtained and compared to previous samples. Also provided herein are methods of reducing anti-EBV IgG levels in the CSF in a subject with MS by administering to the subject autologous T cells (e.g., CTLs) expressing a T cell receptor that specifically binds to an EBV peptide presented on a class I MHC. A reduction in anti-EBV IgG levels in the CSF may be measured by a CSF IgG index. In some implementations, the CSF IgG levels may be calculated by the Reiber and Felgenhauer formula (i.e., see Figure 1). Anti-EBV IgG levels may be reduced by at least 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% following administration of T cells.

In some implementations, the methods comprise improving or stabilizing a symptom (e.g., vision loss, loss of visual acuity, loss or reduction in manual dexterity, increased fatigue, and/or urinary urgency) of MS in a subject, by administering to the subject autologous T cells (e.g., CTLs, such as the peptide specific autologous CTLs described herein) expressing a T cell receptor that specifically binds to an EBV peptide presented on a class I MHC. Also provided herein are methods of improving visual acuity, improving color vision, or stabilizing vision loss in a subject with MS comprising administering to the subject autologous T cells (e.g., CTLs, such as the peptide specific autologous CTLs described herein). In some implementations, provided herein are methods of improving motor skills, balance, or manual dexterity in a subject with MS, comprising administering to the subject autologous T cells described herein. Also provided herein are methods improving sleep in a subject comprising administering to the subject autologous T cells (e.g., CTLs, such as the peptide specific autologous CTLs described herein).

In some implementations, the subject is given a diagnostic test, such as EDSS. In some implementations, the subject is EDSS tested and given an EDSS score prior and/or after T cells administration. The EDSS score may stay the same, or the EDSS score may decrease (e.g., by at least 0.1, 0.2, 0.3, 0.4, 0.5, 0.6, 0.7, 0.8, 0.9, 1.0, 1.5, 2.0, 2.5, 3.0, 3.5, 4.0, 4.5, or 5.0) after T cell administration.

The various methods disclosed herein can be methods for improving walking, vision, balance, cognition, or other symptoms in a subject, such as a subject with multiple sclerosis, and/or methods for improving multiple sclerosis functional composite (MSFC), EDSS, or MSSS scores in a subject, such as a subject with multiple sclerosis. Thus, in certain implementations, the methods of treatment disclosed herein include methods for stabilizing or improving a disability or condition (e.g., motor skills/balance/manual dexterity, sleep, visual acuity or color vision, fatigue, urinary urgency) in a patient, whereby the patient's disability score (as measured by either of these tests or another suitable test) after one week, two weeks, four weeks, 6 weeks, 8 weeks, 10 weeks, three months, six months, one year, or two years of therapy is at least about 1%, at least about 2%, at least about 5%, at least about 10%, at least about 25%, at least about 40%, at least about 50%, or even at least about 60% higher relative to an EDSS score prior to T cell therapy.

For example, a subject's EDSS score can be tested, e.g., assessing the subject's performance on test at different points in time, such as at 0 months (baseline), 1 month, 2 months, 3 months, 6 months, 1 year, and 2 years. In certain implementations, if there is documented decrease in a subject's score after administration of T cells, then the MS is deemed to have stabilized and/or not progressed. In other implementations, if there is neither an increase or decrease in a subject score after administration of T cells, then the MS is deemed to have stabilized and/or not progressed. The EDSS test may be repeated at any point from the start of CTL treatment (e.g., at one week, two weeks three weeks, at four weeks, at one month, two months, three months from the start of CTL treatment) to assess whether the treatment slowed or halted any further worsening in motor skills/balance/manual dexterity, improved sleep, improved visual acuity or color vision, fatigue, urinary urgency.

In some implementations, progression of a walking disability can be tested using a walking test, e.g., assessing the subject's performance on a 25-foot walk test at different points in time. In certain implementations, if there is no documented worsening in walking, then the subject is deemed to have no progressive worsening in walking. For such a patient not already receiving T cell therapy, the subject demonstrating the progressive walking disability commences treatment with T cells, e.g., CTLs. The walking test may be repeated (e.g., at one week, two weeks three weeks, at four weeks, at one month, two months, three months from the start of treatment) to assess whether the treatment slowed or halted any further worsening in walking performance, e.g., as measured by the walking test.

Improvements in cognition outcomes associated with MS therapy, whether slowing of cognitive decline, stabilization of cognitive decline, or improvement of cognitive function, can be assessed using the PASAT (e.g., PASAT 2 or PASAT 3) or SDMT test, or alternatively the MS-COG test (see Erlanger et al., J Neuro Sci 340: 123-129 (2014)).

Actual dosage levels of the active ingredients in the pharmaceutical compositions provided herein may be varied so as to obtain an amount of the active ingredient which is effective to achieve the desired therapeutic response for a particular patient, composition, and mode of administration, without being toxic to the patient.

The selected dosage level will depend upon a variety of factors including the activity of the particular agent employed, the route of administration, the time of administration, the rate of excretion or metabolism of the particular compound being employed, the duration of the treatment, other drugs, compounds and/or materials used in combination with the particular compound employed, the age, sex, weight, condition, general health and prior medical history of the patient being treated, and like factors well known in the medical arts.

### EXEMPLIFICATION:

### Example 1: Study of MS patients and multiple treatments with EBV-specific T cell therapy for MS

Participants donated a 200-400 mL blood sample. Peripheral blood mononuclear cells from this sample were used for laboratory generation of autologous latent membrane protein (LMP1&2)/Epstein-Barr virus nuclear antigen 1 (EBNA1)-specific T cells suspended in clinical grade normal saline. The investigational product is produced by stimulation with gamma-irradiated autologous peripheral blood mononuclear cells infected with the recombinant adenoviral vector AdE1-LMPpoly, an adenoviral vector encoding multiple CD8+ T cell epitopes from EBV nuclear antigen-1 (EBNA1), latent membrane protein 1 (LMP1) and LMP2A . The T cell cultures are then assessed for cell yield, viability and T cell frequency. Approximately 5 weeks will usually pass between collection of the 200-400 mL blood sample and first cell administration.

Each patient received their own T cells stimulated *ex vivo* to enhance reactivity to EBNA1, LMP1 and LMP2A, and was followed through 26 weeks. Patients received the T cell therapy intravenously, at fortnightly intervals. Each dose was given once, the initial dose being 5 × 10⁶ T cells, followed by doses of 1 × 10⁷, 1.5 × 10⁷, and 2 × 10⁷ cells. Four total doses were given over a period of 8 weeks. Each dose of cells was administered via an intravenous line drip, allowing the slow administration of T cells into the blood, rather than a bolus of cells.

Thirteen patients were enrolled. Three patients were withdrawn prior to receiving cell therapy: one for unrelated diagnosis of malignancy, and two for inability to generate EBV-specific T cells. The remaining ten patients each received four T cell infusions per protocol (Table 1, below).

Autologous EBV-CTLs were well tolerated, and no significant adverse events (AE) have been observed. Only one patient experienced a related or probably related AE, which was transient grade 1 "altered taste" judged to be due to DMSO solvent. No grade 4 or 5 AE were reported.

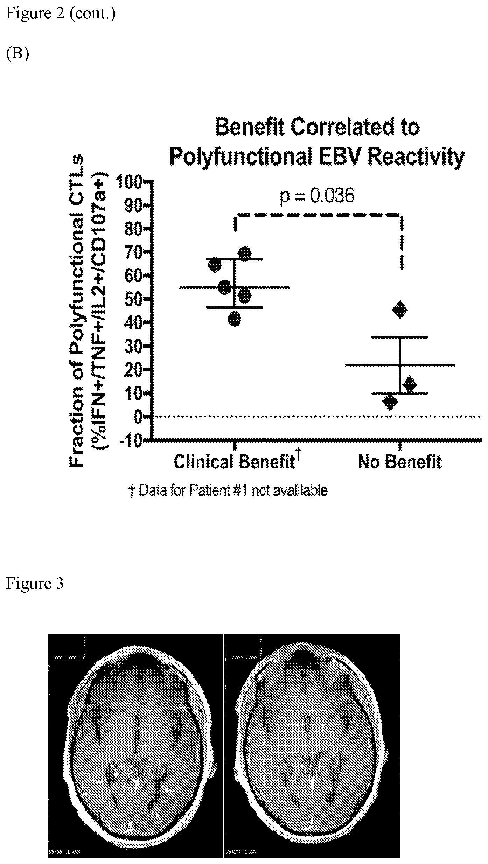

Six patients experienced symptomatic and objective clinical improvement, which commenced 2-14 weeks after the first infusion (Table 1). Reduction in fatigue was a prominent feature in patients with clinical improvement. A correlation between EBV reactivity and clinical response was observed. Patients receiving T cells with higher EBV reactivity experienced greater clinical response. Six patients in the study received T cells with ≥7% EBV reactivity; five of these patients experienced clinical improvement, with 3 patients experiencing improvement in EDSS score. Four patients received T cells with ≤3% EBV reactivity; only one of these had clinical improvement, one had worsening of EDSS, and two reported no change. Clinical improvement during the 6-month study period, or lack thereof, also correlated with biomarkers of EBV-specific T cell function of the administered T cells (Figure 2). Compared to patients without observed benefit, the patients who experienced clinical benefit received therapy that was significantly enriched with EBV-specific CD8+ T cells expressing CD107a, IFNγ and TNFα. In addition, clinical benefit correlated with polyfunctionality of the administered T cells (expression of CD107a, IFNγ, TNFα, and IL-2).

Autologous T cell therapy for MS patients in this study was safe and well tolerated, with no severe AE, and the only AE related to treatment was dysgeusia, likely related to DMSO in the preparation and not the T cells.

The clinical improvements in SPMS and PPMS patients were from a relatively fixed baseline (for up to 5 years) and did not represent resolution of an acute MS flare. Of the 6 patients receiving T cells with EBV reactivity ≥7%, 5 showed clinical improvement, with 3 patients improving their EDSS score.

Consistent with the hypothesized mechanism of action of EBV-specific T cells, there appears to be a dose-response correlation between the EBV reactivity of the T cell product and clinical improvement.

Reduction in fatigue was a consistent and prominent feature in responders. Fatigue is the most disabling symptom of MS and may precede clinical MS onset, often by years.

Our data add to the mounting evidence for a pathogenic role of EBV infection in MS. Because T cells access all CNS compartments, T cell therapy targeting only EBV-infected B cells is a new treatment modality that could offer favorable safety and durable efficacy.

### Example 2: Exemplary method for Multi-parametric Intracellular Cytokine Staining and degranulation analysis of LMP and EBNA1 specific T cells

1. Dilute the LMP/EBNA1 CD8 pepmix (100µg/ml stock), the EBNA1 pepmix (100µg/ml stock) and any HLA-matched peptide epitopes (200µg/mL stock) to be tested to 2µg/mL in RPMI1640-10%FCS (the final concentration in the assay will be 1µg/mL).
2. Dilute the Cell Stimulation Cocktail 1:50 in RPMI1640-10% FCS. (This will be a final dilution of 1:100 in the assay).
   Note: the eBioscience Cell Stimulation Cocktail is stored -20°C following dilution 1 in 5 in RPMI to make a stock concentration of 100x.
3. Add 100µL of the appropriate pepmix, peptide epitope or cell stimulation cocktail, or 100µL RPMI1640-10%FCS (no peptide control) to the appropriate wells of a 96-well V-bottom plate.
4. Dilute PBMC or T cells to 5x10⁶ cells/mL in RPMI1640-10%FCS (this will give 5x10⁵ cells per assay).
5. Add GolgiPlug (Brefeldin A) to cells for a final ratio of 2µL/mL of cells (final concentration of GolgiPlug in the assay will be 1µL/mL).
6. Add GolgiStop (monensin) to cells for a final ratio of 1.4µL/mL of cells (final concentration of GolgiStop in the assay will be 0.7µL/mL).
7. Add FITC conjugated anti-CD107a to cells for a final ratio of 50µL/mL (final amount of anti-CD107a is 5µL/test).
8. Add 100µL of cell suspension per well to the required wells of the 96-well V-bottom plate.
9. Incubate for 4 hours at 37°C/6.5%CO₂.
10. Centrifuge the plate at 2300rpm for 2 minutes.
11. Discard the supernatant. Wash cells by adding 200µL of PBS-2%FCS per well and centrifuge the plate at 2300rpm for 2 minutes. Repeat this step
12. Wash cells twice with PBS-2% FCS 200µL/wash per well, centrifuging the tubes at 1000g (2300 rpm) for 2 min.

### Surface Cell Antigen Staining

13. Resuspend cells in 50µL/well of PBS-2%FCS containing 0.125 of perCP-Cy5.5 conjugated anti-CD8, 0.25µL of Pacific Blue conjugated anti-CD4 and 0.2µL of Live/Dead Near IR. Incubate for 30 minutes at 4°C.
14. Centrifuge the plate at 2300rpm for 2 minutes. Discard the supernatant. Wash the cells by adding 200µL of PBS-2%FCS per well. Centrifuge the plate at 2300rpm for 2 minutes. Repeat this step.
15. Resuspend cells in 100µL per well of BD cytofix/cytoperm solution and incubate at 4°C for 20 minutes.
16. Centrifuge the plate at 2300rpm for 2 minutes. Discard the supernatant. Wash the cells by adding 200µL of BD Perm/Wash per well. Centrifuge the plate at 2300rpm for 2 minutes. Repeat this step.

### Intracellular Cytokine Staining

17. Resuspend fixed/permeabilised cells in 50µL/well of BD Perm/Wash solution containing 1µL of PE-conjugated anti-IL-2, 1µL of AF700 conjugated anti-IFNy and 0.25µL of APC conjugated anti-TNF. Incubate for 30 minutes at 4°C.
18. Centrifuge the plate at 2300rpm for 2 minutes. Discard the supernatant. Wash the cells by adding 200µL of Perm/Wash per well. Centrifuge the plate at 2300rpm for 2 minutes. Repeat this step.
19. Resuspend the cells in 200µL of PBS-2% paraformaldehyde and store at 4°C.
20. Acquire cells using the BD Fortessa. Analyse cytokine production/degranulation using Flow Jo software.

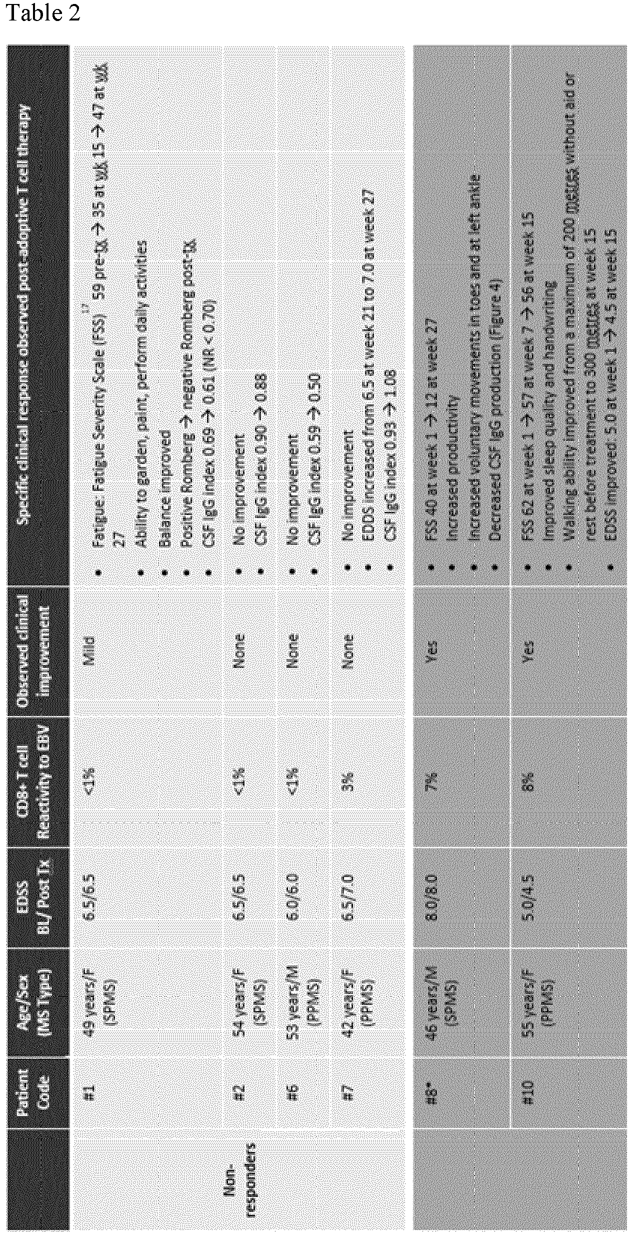

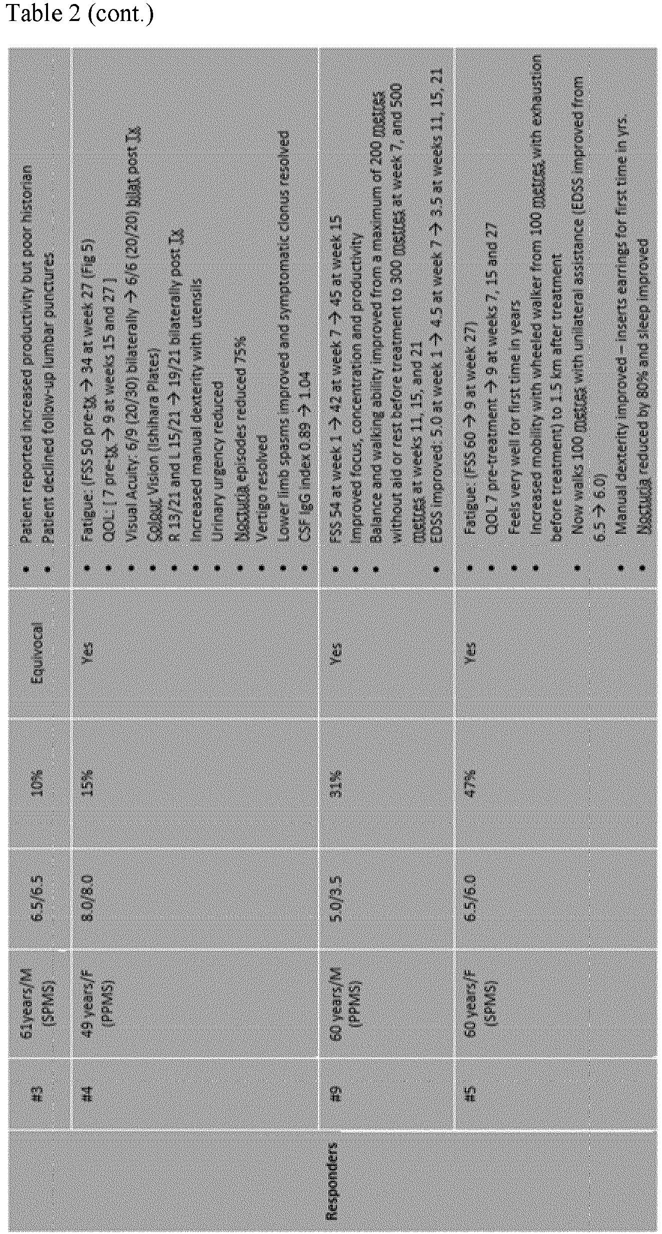

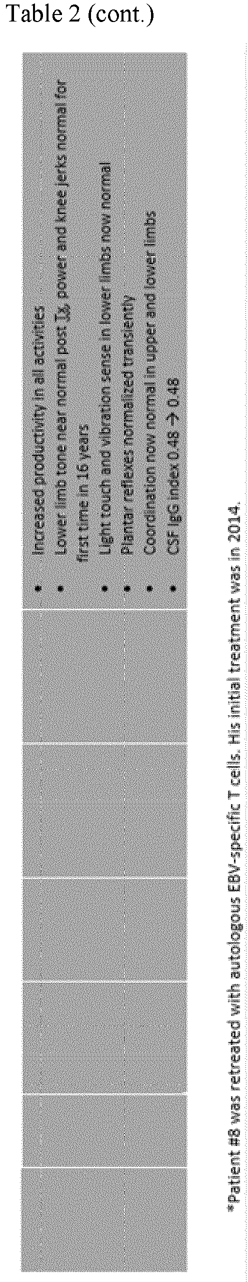

## Claims

1. Autologous cytotoxic T cells (CTLs) expressing a T cell receptor that specifically binds to an EBV peptide presented on a class I MHC for use in treating or preventing secondary progressive multiple sclerosis (MS) or primary progressive MS in a subject, wherein at least 30% of the CTLs express CD107a, IFNγ, TNFa, and IL-2.

2. The CTLs for use of claim 1, wherein at least 40%, at least 50%, or at least 70% of the CTLs express IFNγ, CD107a, IL-2, and TNFa.

3. Autologous cytotoxic T cells (CTLs) for use in treating or preventing secondary progressive multiple sclerosis (MS) or primary progressive MS in a subject, wherein the CTLs are prepared by a method comprising:
a) incubating a sample comprising autologous cytotoxic T cells (CTLs) with antigen-presenting cells (APCs) presenting an EBV peptide, thereby inducing proliferation of peptide-specific T cells in the sample; and
b) analyzing the expression of IFNγ, CD107a, IL-2, and TNFa by the proliferated peptide-specific autologous CTLs;
wherein the peptide-specific autologous CTLs are for administration to the subject if at least 30% of the peptide-specific autologous CTLs express CD107a, IFNγ, TNFa, and IL-2.

4. The CTLs for use of claim 3, wherein the peptide-specific autologous CTLs are for administration to the subject if at least 40%, or at least 50% of the proliferated peptide-specific autologous CTLs express IFNγ, CD107a, IL-2, and TNFa.

5. The CTLs for use of claim 3 or 4, wherein the APCs comprise B cells, antigen-presenting T-cells, dendritic cells, artificial antigen-presenting cells, or aK562 cells.

6. The CTLs for use of any one of claims 1-5, wherein the EBV peptide comprises an amino acid sequence listed in Table 1.

7. The CTLs for use of any one of the preceding claims, comprising administering about 5 × 10⁶ CTLs, about 1 × 10⁷ CTLs, about 1.5 × 10⁷ CTLs, or about 2 × 10⁷ CTLs to the subject in a dose.

## Patentansprüche

1. Autologe zytotoxische T-Zellen (CTLs), die einen T-Zell-Rezeptor exprimieren, der spezifisch an ein EBV-Peptid bindet, das auf einem Klasse-I-MHC präsentiert wird, zur Verwendung in der Behandlung oder Prävention von sekundär progredienter Multipler Sklerose (MS) oder primär progredienter MS in einem Individuum, wobei mindestens 30 % der CTLs CD107a, IFNγ, TNFα und IL-2 exprimieren.

2. CTLs zur Verwendung nach Anspruch 1, wobei mindestens 40 %, mindestens 50 % oder mindestens 70 % der CTLs IFNγ, CD107a, IL-2 und TNFα exprimieren.

3. Autologe zytotoxische T-Zellen (CTLs) zur Verwendung in der Behandlung oder Prävention von sekundär progredienter Multipler Sklerose (MS) oder primär progredienter MS in einem Individuum, wobei die CTLs durch ein Verfahren hergestellt werden, das umfasst:
a) Inkubation einer Probe, die autologe zytotoxische T-Zellen (CTLs) enthält, mit Antigen-präsentierenden Zellen (APCs), die ein EBV-Peptid präsentieren, wodurch die Proliferation peptidspezifischer T-Zellen in der Probe induziert wird; und
b) Analyse der Expression von IFNγ, CD107a, IL-2 und TNFα durch die proliferierten peptidspezifischen autologen CTLs;
wobei die peptidspezifischen autologen CTLs zur Verabreichung an das Subjekt bestimmt sind, wenn mindestens 30 % der peptidspezifischen autologen CTLs CD107a, IFNγ, TNFα und IL-2 exprimieren.

4. CTLs zur Verwendung nach Anspruch 3, wobei die peptidspezifischen autologen CTLs zur Verabreichung an das Subjekt bestimmt sind, wenn mindestens 40 % oder mindestens 50 % der proliferierten peptidspezifischen autologen CTLs IFNγ, CD107a, IL-2 und TNFα exprimieren.

5. CTLs zur Verwendung nach Anspruch 3 oder 4, wobei die APCs B-Zellen, Antigen-präsentierende T-Zellen, dendritische Zellen, künstliche Antigen-präsentierende Zellen oder aK562-Zellen umfassen.

6. CTLs zur Verwendung nach einem der Ansprüche 1-5, wobei das EBV-Peptid eine in Tabelle 1 aufgeführte Aminosäuresequenz umfasst.

7. CTLs zur Verwendung nach einem der vorhergehenden Ansprüche, wobei dem Individuum etwa 5 × 10⁶ CTLs, etwa 1 × 10⁷ CTLs, etwa 1,5 × 10⁷ CTLs oder etwa 2 × 10⁷ CTLs in einer Dosis verabreicht werden.

## Revendications

1. Cellules T cytotoxiques autologues (CTL) exprimant un récepteur de cellules T qui se lie spécifiquement à un peptide EBV présenté sur un CMH de classe I pour une utilisation dans le traitement ou la prévention de la sclérose en plaques (SEP) progressive secondaire ou de la SEP progressive primaire chez un sujet, dans laquelle au moins 30% des CTL expriment CD107a, IFNγ, TNFα, et IL-2.

2. CTL pour une utilisation selon la revendication 1, dans laquelle au moins 40%, au moins 50%, ou au moins 70% des CTL expriment IFNγ, CD107a, IL-2, et TNFα.

3. Cellules T cytotoxiques autologues (CTL) pour une utilisation dans le traitement ou la prévention de la sclérose en plaques (SEP) progressive secondaire ou de la SEP progressive primaire chez un sujet, dans laquelle les CTL sont préparées par un procédé comprenant:
a) incuber un échantillon comprenant des cellules T cytotoxiques autologues (CTL) avec des cellules présentatrices d'antigènes (CPA) présentant un peptide EBV, induisant ainsi la prolifération de cellules T spécifiques du peptide dans l'échantillon; et
b) analyser l'expression de IFNγ, CD107a, IL-2, et TNFα par les CTL autologues spécifiques du peptide proliférées;
dans laquelle les CTL autologues spécifiques du peptide sont pour une administration chez le sujet si au moins 30% des CTL autologues spécifiques du peptide expriment CD107a, IFNγ, TNFα, et IL-2.

4. CTL pour une utilisation selon la revendication 3, dans laquelle les CTL autologues spécifiques du peptide sont pour une administration chez le sujet si au moins 40%, ou au moins 50% des CTL autologues spécifiques du peptide proliférées expriment IFNγ, CD107a, IL-2, et TNFα.

5. CTL pour une utilisation selon la revendication 3 ou 4, dans laquelle les CPA comprennent des cellules B, des cellules T présentatrices d'antigène, des cellules dendritiques, des cellules présentatrices d'antigène artificielles, ou des cellules aK562.

6. CTL pour une utilisation selon l'une quelconque des revendications 1-5, dans laquelle le peptide EBV comprend une séquence d'acides aminés répertoriée dans le Tableau 1.

7. CTL pour une utilisation selon l'une quelconque des revendications précédentes, comprenant l'administration d'environ 5 x 10⁶ CTL, d'environ 1 x 10⁷ CTL, d'environ 1,5 x 10⁷ CTL, ou d'environ 2 x 10⁷ CTL au sujet dans une dose.
